## Europäisches Patentamt
## European Patent Office
## Office européen des brevets

(11) Publication number: **0 172 381**
**B1**

(12) **EUROPEAN PATENT SPECIFICATION**

(45) Date of publication of patent specification: **13.06.90**

(51) Int. Cl.⁵: **A 61 M 5/14**

(21) Application number: **85108608.2**

(22) Date of filing: **10.07.85**

(54) **Clamp for regulating the flow of a liquid.**

(30) Priority: **11.07.84 ES 280599 u**

(43) Date of publication of application:
**26.02.86 Bulletin 86/09**

(45) Publication of the grant of the patent:
**13.06.90 Bulletin 90/24**

(84) Designated Contracting States:
**AT BE CH DE FR GB IT LI LU NL SE**

(56) References cited:
**DE-A-3 104 373**
**US-A-1 804 825**
**US-A-3 544 060**
**US-A-3 584 830**
**US-A-3 948 477**
**US-A-4 139 017**

(73) Proprietor: **Lopez Torras, Manuel**
**Torras i Bages, 23**
**Cornella Barcelona (ES)**

(72) Inventor: **Lopez Torras, Manuel**
**Torras i Bages, 23**
**Cornella Barcelona (ES)**

(74) Representative: **Vossius & Partner**
**Siebertstrasse 4 P.O. Box 86 07 67**
**D-8000 München 86 (DE)**

# Description

This patent relates to a clamp for regulating the flow of a liquid through a flexible conduit, it being particularly, although not exclusively, used for controlling medicinal liquids.

Therapy by infusion, generally intravenous, of liquids of various natures, such as sera having different components, blood plasma or the like, etc., to patients requiring such measures is a regular medical practice.

The known infusion equipment, generally of the so-called disposable type, basically comprises a flexible liquid glow conduit having at one end a cone for insertion of the injection needle and a transparent dripper device at the other end, allowing the drop-per-minute rate to be observed and controlled. This dripper device is sharpened at the end thereof to facilitate perforation of the plug of the bag or bottle containing the liquid to be perfused. Under the dripper device there is inserted an adjustable throttle device, usually providing uncertain results with respect to the exact metering of the liquid and ease of adjustment.

The above mentioned infusion equipment is situated beside the hospitalised patient, with the bottle hanging from a support and extended by a conduit to the patient's body. Movements of the patient's arm, possible contacts by third persons with the conduit, handling of the flow throttle by the medical personnel to adjust the flow rate and the swinging motion of the bottle itself all cause oscillatory movements of the whole structure, which is subjected to knocks unnecessarily upsetting the drop per minute rate. This is extremely important, if it is considered that the perfusion liquids generally contain medicinal drugs such as antibiotics, heart tonics, diuretics, cortisones and others, which require to be metered as accurately and regularly as possible.

From US—A—3 948 477 a screw clamp comprising the features of the preamble of claim 1 is known for flexible tubing including a handle having a screw housing attached to one end thereof. A screw is threaded in screw housing and can be rotated to clamp flexible tubing against a portion of screw housing, thereby selectively preventing the passage of fluid through the tubing. The handle is adapted to be gripped by the middle, ring and small fingers of the hand so that the screw can be rotated with the thumb and forefinger of the same hand to clamp the flexible tube against the base of the screw housing. This known device does not comprise any mounting means by which the screw clamp may be attached to any stationary structural member.

A further screw clamp is described in US—A—3 584 830. The clamp includes a pair of rigid jaws mounted for movement towards and away from each other, one of the jaws having a substantially semicylindrical channel and the other jaw having an elongated and arcuate projection of generally semi-circular cross section which in both longitudinal and transverse dimensions than the channel. A knob carried by a threaded post secured to one of the jaws may be rotated to force the jaws together for clamping and collapsing a tube of resilient plastic or other suitable material extending therebetween. This known screw clamp does also not comprise any means for attaching it to a fixed support.

The features of the liquid flow regulating clamp according to the present invention are indicated in the claim.

The regulating clamp of this application removes the aforesaid drawbacks and provides functional advantages guaranteeing, on the one hand, gentle, adjustable control of the drop rate and, on the other, complete immobilisation of the infusion equipment in the part thereof extending from the bottle or container of the liquid to be perfused to the lower portion of the dripper device, which normally prevents any knocks or movement in the said length of the conduit and of the infusion equipment and the resulting upsetting of the drip rate.

To facilitate the explanation, there are attached to the specification drawings which as a nonlimiting example show one embodiment of a flow regulating clamp for liquids, according to the principles stated in the claims.

In the drawings:

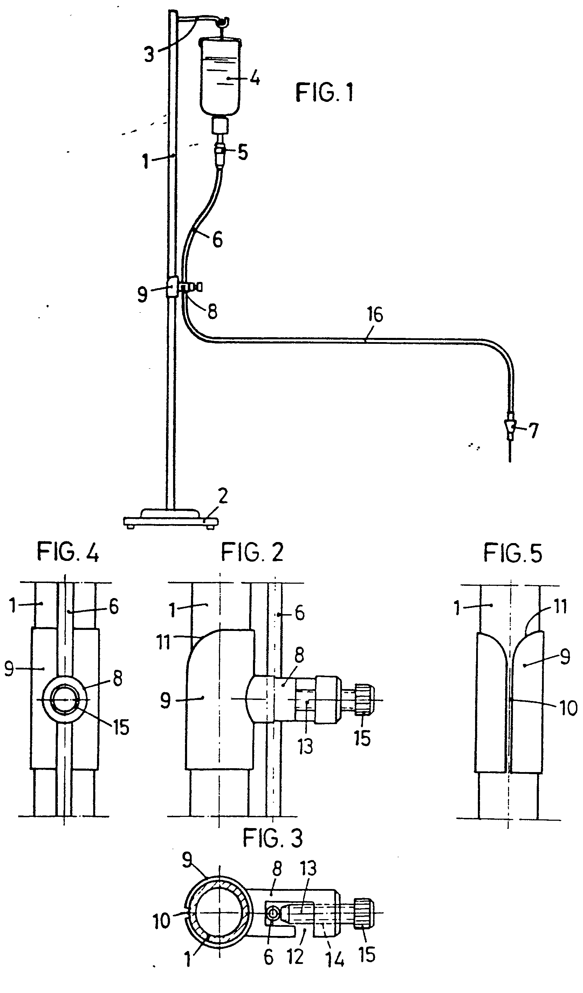

Figure 1 illustrates a physiological liquid transfusion equipment, with the outlet conduit towards a patient's body and being provided with a regulating clamp according to the invention;

Figure 2 is a side view of a detail of the new regulating device, which is illustrated in plan view in Figure 3 and in side elevation in Figures 4 and 5.

The parts designated with the numbers in the drawings correspond to the parts listed hereinafter.

The equipment for administration of physiological liquids to the human body comprises a support 1 having a base 2 and an arm 3 for holding the bottle 4 containing the liquid to be administered, asociated with a dripper device connected at 5 to the conduit 6, the other end of which is provided with a cone 7 for connection to the injection needle for the immediate administration of the liquid to a patient's body.

The device being described comprises as outstanding feature a central body 8 advantageously having a cylindrical shape, one of the ends of which is extended by an also tubular cylindrical body 9 defining two semi-cylindrical wings which will be applied as a prehensile grip to the support 1, of varying diameter, of the equipment. The confronting edges of the extensions, which define a separable area 10, have at one end the rounded and divergent edges 11, to facilitate engagement of the device with the support 1.

On the middle side portion thereof, the body 8 is provided with an L-shaped recess 12 for receiving sideways and locating in the end of the L the conduit 6, which will be retained in the body 8 by the stud 13 provided with a fine pitch thread mating with the thread 14 in the axial bore of the body 8 at the end thereof. The head 15 of the stem, advantageously knurled on the side surface thereof, will facilitate the holding and operation of said stem and the consequent retention of the conduit 6.

On continuing to rotate the control or head 15, the flexible tube forming the conduit 6 is flattened to a greater or lesser extent and, thereby, an effective adjustment of the passage for the liquid is provided. As described, it is understood that the part of the conduit 6 comprised between the clamp 8 and the bottle 4 will be held immobile, in spite of the lower portion 16 of the conduit being affected by oscillations or other more or less sharp movements made by the patient or other circumstances. This would not affect the integrity of the assembly and the equipment will be always in conditions to operate troublefree. Likewise, the control of the flow through the conduit 6 will be very accurate by way of the above described screw device, and any adjustment of the drop rate being required may be obtained. A further advantage of the device is the possibility of inserting and removing the conduit 6 into and from the body 8 through the side of the latter in any point thereof.

This feature is important, since it provides a greater ease for the assembly of infusion equipment, preventing what is a necessary step in the known equipment, i.e. the prior insertion of the conduit through the flow regulator, before connecting the tube to the dripper device and with the needle holder cone.

All that does not affect, alter, change or modify the essence of the clamp described, will be variable for the purposes hereof.

## Claim

A liquid flow regulating clamp, comprising a solid support body (8), provided at the side thereof with a recess (12) for allowing insertion of a flow conduit tube (6) for the liquid to be controlled and the retention thereof in one end of the recess, by the termination of a threaded stem (13) engaged in one end of the holding body (8), while the other end is fixedly attached to a prehensile member, characterized in that the prehensile member is formed by a circular cylinder (9) provided at the side thereof remote from the support with an opening extending the whole of the length of the cylinder so as to form resilient walls for engagement with a vertical support member (1), at least one of the walls of the cylinder at one end of the opening being rounded for facilitating its attachment to the circular support of a unit.

## Patentanspruch

Klemme zur Regelung einer flüssigkeitsströmung mit einem festen Stützkörper (8), der an seiner Seite mit einer Ausnehmung (12) ausgebildet ist, so daß es möglich ist, ein Flüssigkeitsleitungsrohr (6) für die zu regelnde Flüssigkeit einzusetzen und an einem Ende der Ausnehmung festzuhalten mittels dem Ende eines mit einem Gewinde versehenen Bolzen (13), der mit einem Ende des Haltekörpers (8) im Eingriff steht, während das andere Ende fest an einem Griffelement angebracht ist, dadurch gekennzeichnet, daß das Griffelement von einem kreisförmigen Zylinder (9) gebildet wird, der an seiner Seite entfernt von der Stütze mit einer Öffnung ausgebildet ist, die sich über die gesamte Länge des Zylinders erstreckt, so daß elastische Wände zum in Eingriff nehmen mit einem senkrechten Stützelement (1) gebildet werden, wobei mindestens eine der Wände des Zylinders an einem Ende der Öffnung abgerundet ist, so daß seine Anbringung an der kreisförmigen Stütze einer Einheit erleichtert wird.

## Revendication

Pince pour la régulation de l'écoulement d'un liquide, comprenant un corps de support plein (8), pourvu sur son côté d'une encoche (12) pour permettre l'introduction d'une conduite tubulaire d'écoulement (6) pour le liquide devant être contrôlé, et sa rétention dans une extrémité de l'encoche, par le bout d'une tige vissée (13) introduite dans une des extrémités du support (8) tandis que l'autre extrémité est fixée à un dispositif de préhension, caractérisée en ce que, le dispositif de préhension est formé par un cylindre circulaire (9) pouvru sur son côté éloigné de support d'une ouverture s'étendant tout le long de cylindre de sorte qu'il forme des parois extensibles pour être monté sur une barre de support verticale (1), au moins une des parois du cylindre étant arrondie à l'une des extrémités de l'ouverture pour faciliter sa fixation au support circulaire d'une unité.

FIG. 1

FIG. 4

FIG. 2

FIG. 5

FIG. 3